# EUROPEAN PATENT SPECIFICATION

(11) **EP 0 368 931 B1**
(45) Date of publication and mention of the grant of the patent: **13.10.1993**
(21) Application number: 88907872.1
(22) Date of filing: 04.08.1988
(51) Int. Cl.: A61M 29/00

(54) **VALVED CATHETER DEVICE**
KATHETER MIT VENTIL
CATHETER A VALVE

(30) Priority: 07.08.1987 US 83624; 01.08.1988 US 226674
(43) Date of publication of application: 23.05.1990
(73) Proprietor: TARGET THERAPEUTICS, INC., Fremont, CA 94537-5120 (US)
(72) Inventor: ENGELSON, Erik, T., Palo Alta, CA 94303 (US); DANIELS, John, R., Pacific Palisades, CA 90272 (US)
(74) Representative: Senior, Alan Murray
(86) International application number: US8802656
(87) International publication number: WO8901352

(56) References cited:
- DE-A- 3 408 809
- US-A- 3 402 717
- US-A- 4 646 742

## Description

This invention relates to catheter devices and methods for accessing internal body target sites along a small-vessel path.

Catheters are being used increasingly as a means for delivering diagnostic or therapeutic agents to internal target sites, and to perform mechanical functions on vasculatures that can be accessed through the circulatory system. For example, in angiography, catheters are designed to deliver a radiopaque agent to a target site within a blood vessel, to allow radiographic viewing of the vessel and blood flow characteristics near the release site. For the treatment of localized disease, such as solid tumors, catheters allow therapeutic agents to be delivered to the target site at a relatively high concentration, with reduction in drug delivery to non-target sites. Often the target site which one wishes to access is in a tissue, such as brain, liver or kidney, which requires catheter placement along a tortuous path through small vessels or ducts, such as arterial vessels or biliary ducts. Typically, the vessel path will include vessel branch points at which the path may follow either a relatively larger-diameter, higher-flow branch vessel, or a relatively smaller, lower-flow branch vessel.

Heretofore, three general types of catheters have been developed for accessing internal target sites. One type is a torqueable catheter having relatively rigid tube construction and large-diameter lumen. In particular, the catheter tube may be formed as a braided fiber or wire laminate which has high torque properties. The distal portion of the catheter can be made narrower and more flexible by eliminating laminate windings or braid from this portion of the catheter, but this compromises torque transmission. Torqueable catheters of this type are generally too large in diameter and too rigid to be safely advanced through narrow, tortuous vessel or duct paths.

Another type of guidable catheter is a guide-wire catheter which contains a single-lumen catheter used in conjunction with a flexible, torqueable, guidewire which can be moved axially within the catheter. In a typical catheter-placement operation, the wire is advanced along the vessel pathway, using wire torquing to orient the bent tip of the wire along the selected path, i.e., into and through selected branch vessels and/or regions of sharp bends. The catheter is then advanced along the wire with the wire held in place. The wire and catheter are alternately advanced in this manner until the target site is reached. Thereafter, the wire can be removed to allow fluid delivery through the catheter into the site. Since the wire can be both torqueable and quite flexible, and the catheter can be a thin-walled flexible tube, the catheter device is well suited for accessing sites via small-diameter tortuous paths.

Another general class of guidable catheters have a distal-end balloon which can be partially inflated to carry the catheter in the direction of highest blood flow, and therefore along a vessel path having maximum blood flow. The balloon may be further inflated, at a selected target site, for purposes of occluding blood flow, or for anchoring the catheter end at the selected site. Extending the balloon to contact the walls of a blood vessel can also be useful in relaxing spasmodic vessel muscles, resulting in less vessel constriction. Balloon catheters thus have the advantage over guide-wire catheters in that they can take advantage of blood flow for advancing along a vessel pathway, and various advantages relating to balloon contact with vessel walls can be achieved.

One type of balloon catheter has a double-lumen construction, where one lumen communicates with the distal balloon, for transferring fluid to or from the balloon. The second lumen allows delivery of injected material, such as a radio-opaque tracer material or therapeutic agent, into the target site. One advantage of the double-lumen catheter is the ability to inflate the balloon to relatively high pressure, which is particularly useful when the balloon is used for stretching a vessel wall, in a catheter treatment for removing vessel plaque. Also, the catheter can be firmly anchored at the target site when the balloon is in a highly inflated state. The double-lumen balloon catheter, however, is not well suited for guidance along small-diameter, tortuous pathways, since the catheter typically has a relatively large outer shaft diameter, and these shafts are generally relatively inflexible. Alternatively, the two catheter lumens may be made relatively small, but here fluid passage through the lumens is slow and limited to low-viscosity agents. Also, since the catheter is guided by blood flow, the device is limited in use to vessel paths with highest blood flow.

In a second balloon-catheter construction, the catheter has a single-lumen tube which communicates with a slow-leak balloon at the distal tube end. In operation, fluid is supplied through the tube at a slow controlled rate, to maintain the balloon at an inflated condition which promotes fluid-directed movement through the vessel path. The single lumen tube can have a small-diameter, highly flexible construction which permits movement along a small-diameter, tortuous vessel path. The ability to guide the catheter, however, is limited to vessel or duct branches with greater flow, as above, so the catheter is not generally useful for accessing a site against the direction of flow, or along a pathway which includes relatively low-flow branches. Another limitation of the single-lumen catheter is that the slow-leak principle of balloon inflation does not allow for high-flow delivery of fluid material or delivery of particulate suspension at the target site.

A third balloon catheter construction has a single-lumen catheter which communicates with a sealed balloon. The catheter is able to access small-vessel tortuous paths and allows relatively high balloon inflation pressures. The catheter is limited, however, to vessel paths of highest blood flow, and of course cannot be used to deliver fluid to the target site.

### 3. Summary of the Invention

It is a general object of the invention to provide an improved catheter device for accessing a target site along a small-vessel tortuous path.

A related object of the invention is to provide such a device which overcomes or reduces above-discussed problems and limitations associated with prior art guidable catheters.

A more specific object of the invention is to combine advantages of balloon catheters and guide-wire catheters in a single device.

US-A-4 646 742, upon which the prior art portion of claim 1 is based, discloses a modified form of the double lumen balloon catheter construction referred to above in that two coaxial tubes are provided with the inner tube being axially movable between aperture closing and aperture opening positions with balloon inflating fluid being conducted in the annular space between the tubes when the inner tube is in its aperture-closing position. The guidewire is passed along the inner tube. The present invention as defined in claim 1, provides a less bulky and simpler structure more suited to be passed along fine vessels in that no inner axially movable tube is required. Control of inflation of the balloon and of the direction of the feed in the catheter is achieved solely by means of the guidewire which, as well as being rotatable for guiding the tip of the catheter is also usable as a valve means in conjunction with the aperture to control inflation of the balloon, the aperture itself being defined by an annular ring mounted in the distal end of the catheter.

The balloon may be carried at the distal end of catheter or along a distal end section of a catheter tube, or formed as an intermediate inflatable section of a catheter tube.

The guidewire and catheter aperture define a valve structure effective to block the aperture at one or more selected wire portions, to permit the catheter balloon to be inflated by supplying fluid through the catheter lumen. The valve structure may be designed for blocking the valve structure partially and/or completely.

In one general embodiment, the ring aperture is dimensioned to receive a distal end segment of the guidewire freely therethrough, with limited wire clearance. Where the catheter is formed of a tube having an inflatable distal end balloon, the ring is positioned at the distal end of the balloon. Where the balloon is disposed along an end section of the catheter tube, the ring is disposed distal to the balloon end section or distal to an opening communicating the catheter tube with the balloon. The guidewire may additionally include a radial enlargement which, when positioned against the annular ring, acts to block the ring aperture completely.

The catheter can be used for accessing an internal body site along a narrow-vessel tortuous path which includes some branch points in which the path follows the larger-diameter of two branch vessels, and some in which the path follows the smaller diameter of two branch vessels.

The guidewire and catheter are advanced along the vessel pathway toward the target site. When a branch point at which the vessel path follows the larger-diameter of two branch vessels is reached, the guidewire is placed in a position to partially (or completely) close the balloon valve, and the balloon is inflated, allowing the distal end of the catheter to be carried by fluid flow into lager-diameter vessel. When a branch point at which the vessel path follows the smaller diameter of two branch vessels is reached, the guidewire can be torqued to orient the guidewire tip in the direction of the smaller-diameter vessel and the catheter then advanced into the smaller vessel, with the catheter balloon preferably in an uninflated state.

The catheter can also be manipulated for balloon inflation at a position along a vessel pathway where vessel constriction is encountered, or to anchor the catheter at the target site.

These and other objects and features of the invention will become more fully apparent when the following detailed description of the invention is read in conjunction with the accompanying drawings.

### Brief Description of the Drawings

Figure 1 shows a catheter device constructed according to one embodiment of the invention;
Figure 2 is an enlarged sectional view taken along line 2-2 of Figure 1;
Figure 3A shows the enlarged distal end section of the Figure 2 catheter device with the balloon in an inflated condition;
Figure 3B is an enlarged, fragmentary view of a modified valve structure;
Figures 4 and 5 are enlarged sectional views of the distal end sections of catheter devices constructed according to various alternative embodiments of the invention;
Figure 6 shows a portion of a small-vessel tortuous path in a target tissue;
Figure 7 shows a branch junction region from Figure 6, in which the path of the catheter follows the larger-diameter of two branch vessels; and
Figure 8 shows a branch junction region from Figure 6, in which the path of the catheter follows the smaller-diameter of two branch vessels.

### Detailed Description of the Invention

### I. Catheter Device Construction

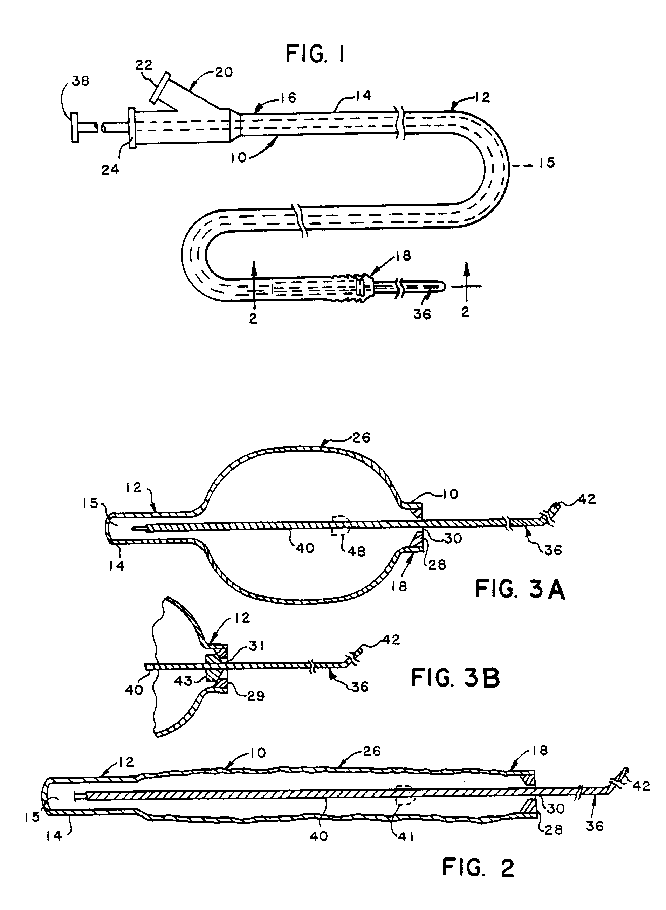

Figures 1-3 show a catheter device 10 constructed according to one embodiment of the invention. Device 10 includes a catheter 12 composed of a flexible, thin-walled tube 14 having an inner lumen 15 extending between proximal and distal catheter end regions 16, 18, respectively. The proximal catheter end is provided with a syringe fitting 20 through which fluid can be supplied to the catheter lumen through a port 22. The fitting includes an axially extending port 24 also communicating with the catheter's inner lumen. Tube 14 preferably has an inner diameter of approximately 0.38-1.52 mm (15-60 mils) and walls that are approximately 0.076-0.38 mm (3-15 mils) thick. The total tube length is preferably between about 50-300 cm.

With reference now to Figures 2 and 3, the distal end of the catheter is provided with an inflatable balloon 26 which forms an intermediate section of the distal end region of tube 14. The balloon is preferably about 0.5 to 2 cm in length, and has a wall section which can be inflated by fluid supply through the catheter lumen, when the distal end of the tube is partially blocked in a manner to be described below. The balloon wall section is preferably formed integrally with the tube, according to known extrusion methods for producing a thin walled-extruded tube with a more flexible distal-end wall section. In particular, the balloon may be formed by inflating the balloon section in a heated condition, then deflating when the balloon wall section has cooled. Alternatively, the balloon may be formed from a sleeve of elastomeric material, such as silicon rubber, and attached at its opposite sleeve ends to relatively more rigid tube sections. The region along the length of the balloon is also referred to herein as means communicating the catheter lumen with the balloon. The catheter tube adjacent the balloon is preferably provided with a radio-opaque band for X-ray imaging during use.

Disposed in the distal end of the catheter tube is an annular plug or ring 28 defining an aperture 30 formed axially in the ring. The ring, which is also referred to herein as means defining aperture 30, is positioned downstream of (on the distal side of) the means communicating the catheter lumen with the inflatable balloon, i.e., distal with respect to the balloon. The ring has an inwardly tapered, frustoconical wall section which acts to guide the guidewire through the central aperture in the ring. The diameter of the aperture is typically about 40-80% of the lumen diameter. The ring may be made of a suitable metallic or nonmetallic material, and can be attached to the catheter tube by heat shrinking, solvent bonding or the like.

The catheter device also includes an elongate, torqueable guidewire 36 which is constructed to extend through the catheter for axial sliding therein. The length of the guidewire is typically at least about 10-50 cm longer than the catheter, such that the distal end of the guidewire, seen in Figures 2 and 3, can be extended at least several centimeters beyond the distal end of the catheter, while allowing the proximal end of the wire to be manipulated, such as by torquing, adjacent the proximal end of the catheter. The proximal end of the wire is equipped with a handle 38, such as a pin vice, for applying torque to the wire during a catheter operation.

The guidewire may have a variable or step diameter along its length, typically including a larger-diameter, stiffer proximal region, and one or more smaller-diameter, more flexible distal end regions, giving the wire good torqueability in its more proximal region, and better flexibility and maneuverability along its more distal region where the wire is advanced along small-diameter tortuous pathways. Typical wire dimensions, for a catheter having an lumen diameter of between about 0.51-1.27 mm (20-50 mils) are a proximal segment extending along all but the last 20-50 cm of wire and having a diameter of between about 0.46-1.02 mm (18-40 mils), and one or more reduced diameter segments 20-50 cm in length having diameters of between about 0.20-0.46 mm (8-18 mils).

In addition the distal end portion of the wire may have a substantially constant taper, down to a final wire thickness of about 0.025-0.13 mm (1-5 mils), greater distal-end flexibility. This tapered region is preferably encased in a constant-diameter platinum coil, such as coil 40 seen in Figures 2 and 3. The guidewire terminates in a bent tip 42 which can be oriented by torquing.

The guidewire is preferably made of stainless steel such as is commercially available from Wytech or National Standard. The tapered tip may be made by a suitable technique, such as by grinding. In the embodiment shown, guidewire 36 is 0.20-0.51 mm (8-20 mils) at its proximal end and tapers down to a 0.05 mm (2 mil) distal tip over about a 10-20 cm length. The coil is made conventionally of wound 0.076 mm (3 mil) platinum, tungsten or other suitably radiopaque wire commercially available, e.g., from California Fine Wire Company or Sigmund Cohn. This coil preferably has an inner diameter of 0.18 mm (7 mils) and a length of approximately 2-20 cm. The coil is attached to guidewire 12 by an appropriate technique, such as soldering or brazing.

The diameter of the distal end section of the wire, including wire coil 40, is preferably about 0.013-0.05 mm (0.5-2 mils) less than that of aperture 30, allowing a distal end portion of the wire to be moved freely (with clearance) through the aperture. At the same time, the limited clearance between the guidewire and aperture forms a valve structure which is effective to partially block the aperture, such that fluid supplied through the catheter will inflate the catheter balloon. The limited clearance between the guidewire is selected so as to allow the balloon to be inflated to a greater (smaller clearance) or lesser (larger clearance) balloon pressure, and/or to allow a desired leakage rate from the balloon in the inflated state.

The guidewire may optionally include an axial enlargement, such as shown in dotted lines at 41 in Figure 2, which is effective to completely block the catheter aperture, when the guidewire is moved to position the enlargement against the aperture ring. This modification is useful where it is desired to inflate the balloon to a relatively high pressure, such as where the catheter device is used for stretching a vessel for purposes of plaque compression.

Figure 3B shows a modification of the Figure 3A valve structure. The modified catheter device, like device 10 shown in Figure 3A, includes a catheter tube 12, a balloon 26, and a guidewire 36. An annular plug or ring 29 disposed at the axial end of the catheter tube defines an aperture 31 whose diameter is greater than that of aperture 30 in device 10. In particular, the clearance between the aperture and the guidewire is such as to allow relatively free flow of fluid through the catheter, with the wire in the position shown in the figure. The portion of the ring defining the aperture is tapered at its inwardly facing side, as shown.

The Figure 3B valve structure further includes a radial enlargement 43 carried on the guidewire. The enlargement is dimensioned to move freely through tube 12 and to form a sealed valve closure with ring 29, when the wire is advanced to place the enlargement against the ring. The outer edge of the enlargement is tapered inwardly, as shown, to center the enlargement on the ring for sealing. The reduced-diameter aperture in the valve structure could alternatively be formed by a smaller-diameter catheter tube section, distal to the balloon.

The valve structure just described allows the catheter balloon to be inflated to greater pressure by virtue of complete valve closure. The device also allows for relatively high fluid delivery through the catheter with the guidewire positioned such that the wire enlargement is within the catheter balloon, as shown.

In operation, the guidewire in device 10, or in the Figure 3B embodiment, is placed in the catheter through port 24 in fitting 20, and threaded through the catheter until the wire's distal end extends from the distal end of the catheter, as shown in Figure 1. During a catheter placement operation, as will be described in Section II below, it will be advantageous to operate the catheter in an uninflated condition during some phases of operation, and in an inflated condition at other times. To inflate the balloon, in the Figure 3A device, the guidewire is moved axially into and through aperture 30, to partially block the valve aperture, and fluid is supplied through the lumen through port 22 in fitting 20, acting to fill and inflate the balloon. Once the balloon is inflated, material is supplied through the catheter under slight pressure to offset fluid leakage through the partially blocked valve. To inflate the balloon in the Figure 3B embodiment, the guidewire is first positioned for complete valve closure.

To deflate the balloon, the guidewire is retracted, to unblock the aperture and cause the fluid in the balloon to leak out the distal end of the catheter, with progressive balloon deflation. Alternatively, the balloon can be deflated by withdrawing fluid from the catheter through port 22.

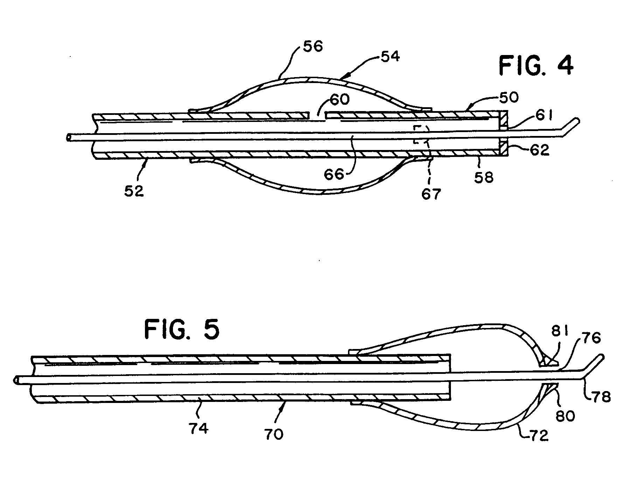

Figure 4 illustrates a catheter device 50 in which the catheter, indicated at 52, has an inflatable balloon 54 which is formed by an inflatable sleeve 56 secured at its opposite ends to a thin-walled, flexible catheter tube 58. The balloon sleeve may be formed of a thin polymer material, and preferably an elastomeric, stretchable material, such as silicon or latex rubber, or alternatively, a non-stretchable film material, such as polyethylene. Attachment of the sleeve ends to the catheter tube is made by gluing, heat sealing, or the like, also according to known methods. The advantage of an elastomeric sleeve is that it tends to remain flush with the tube in an uninflated state, and also tends to resist balloon inflation. Therefore, the balloon will tend to deflate itself when fluid pressure in the tube is released.

An opening 60 formed in the catheter tube provides means communicating the balloon with the catheter lumen. Distal to this opening is a ring 62, which defines an aperture 61 in the catheter tube. A guidewire 66 in the device has an outer diameter which, as above, is dimensioned to partially block the aperture when the wire is moved through the ring. This wire thus forms with the ring valve structure for partially blocking the catheter lumen downstream of opening 60. The guidewire may have substantially the same construction as guidewire 36 in device 10. The operation of device 50, in balloon inflating and deflating operations, is substantially identical to that of device 10.

The guidewire in device 50 may be additionally provided with a radial enlargement, shown in dotted lines at 67 in Figure 4. This enlargement is dimensioned to allow free guidewire movement through the catheter tube, and to form a sealed valve closure with ring 62, when the wire is advanced to place the enlargement against the ring. Like the device shown in Figure 3B, the ring in device 50 may have a greater clearance with the guidewire, to facilitate fluid flow through the catheter, with the guidewire positioned as shown, when the guidewire is provided with a radial enlargement for complete valve closure. That is, the device may be designed for partial and/or complete valve closure.

Figure 5 shows a similar type of catheter device 70 having a distal-end balloon 72 which is attached to and extends from the distal end of the catheter tube, indicated at 74. The distal end of the catheter thus provides means communicating the balloon with the catheter lumen. The balloon is formed of a membranous or elastomeric sac having a distal opening 76 which is dimensioned to receive the distal end of a guidewire 78 freely therethrough, with limited clearance. The opening is reinforced by a plug 80 whose inner bore with the same diameter as opening 76. This plug, which is preferably a flexible elastomeric material, may be formed integrally with the balloon, or attached to the balloon as by gluing. The opening and plug form a ring which defines a central aperture 81 in the catheter.

Guidewire 78 in the device has an outer diameter which is dimensioned to be received through aperture 81, to partially block the lumen of the catheter tube, when the wire is moved through the tube. The wire and aperture formed in the distal end of the balloon collectively form valve structure for use in inflating the balloon, as above. Where it is desired to produce complete valve closure, the guidewire may be provided with a radial enlargement as above. Alternatively, the opening at the distal end of the balloon can be dimensioned to form an elastomeric seal with the guidewire.

### II. Catheter Accessing Method

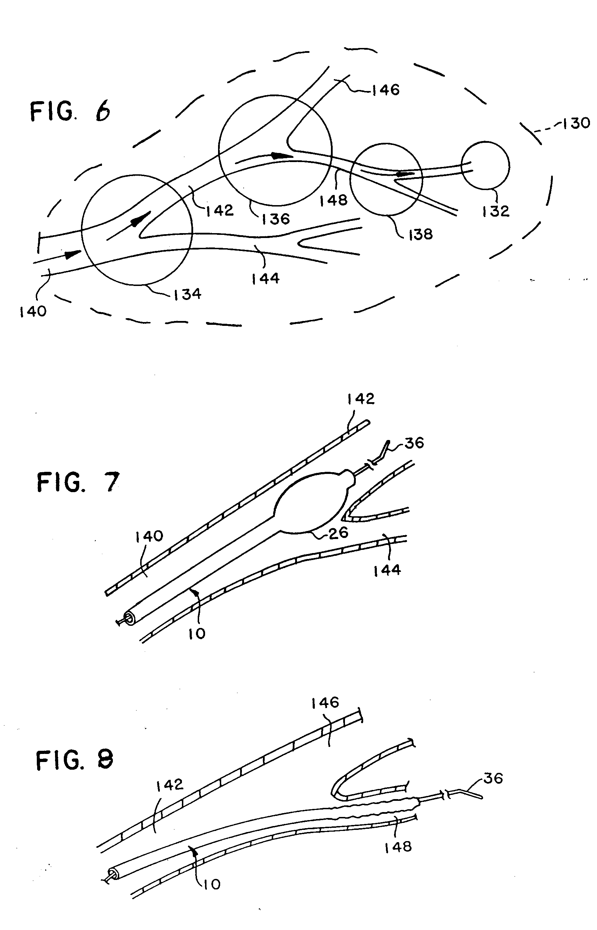

The method of accessing an internal body site along a small-vessel, tortuous vessel or duct path, using the above described catheter, will now be described with reference to Figures 6-8. Figure 6 shows a region of soft tissue 130, such as brain or liver tissue, containing a target site 132 which is to be accessed. The small-vessel path within this tissue region which leads to the target site is shown by arrows in the figure and includes three branch points, indicated at 134, 136, and 138, all supplied by a trunk vessel 140. Each branch point diverges into two branch vessels, such as branch vessels 142 and 144, diverging from vessel 140, and branch vessels 146 and 148 diverging from vessel 142. Often at a branch point along the vessel path, one of the two branch vessels, such as vessels 142 and 146, will have a relatively larger diameter and greater blood volume flow than the other branch vessel, such as vessels 144 and 148.

The method employs a guidewire catheter device of the type described above in which an inner-lumen balloon catheter and a guidewire movable therein define a valve structure which can be manipulated to block the distal end of the catheter, for purposes of supplying fluid to and inflating the balloon. In a typical operation involving a target site accessed through a vascular system, the catheter device is threaded as a unit from an external access site through the vasculature to a region adjacent, but not into the tortuous path region of the target tissue. This is done, in the usual case where the catheter must pass through the cardiac aorta, by first placing a relatively large diameter guiding catheter (e.g., about 1.0 mm (40 mils) inner diameter) from the access site through the aorta and toward the target site. The present catheter and guidewire are then threaded through the guiding catheter past the aorta, where large-vessel diameters and high blood flow volumes make it difficult or impossible to control the movement and position of the catheter. Once beyond the guiding catheter, the catheter and guidewire can be independently controlled to move toward the target site. In general, the path from the access site to the region adjacent the tissue is easily accessible, in that sharp bends, small-lumen vessels, and or soft tissue structure are not encountered.

The distal end of the device is now advanced into the target tissue along the selected path until branch points in the vessels or ducts making up the small-vessel path are encountered, as illustrated in Figure 6. For purposes of illustration, it is assumed that the catheter has been moved along the path until branch point 134 in Figure 6 is encountered. Since the selected path follows the larger-diameter, greater-flow branch vessel 142, the catheter can be guided by blood flow, by inflating the catheter balloon and allowing the distal catheter end to flow into the larger vessel, as illustrated in Figure 7, which shows the catheter device being guided into and through vessel 142. The balloon is inflated, before the branch point is reached by the procedure mentioned above, which involves first moving the guidewire in the device to block the catheter aperture, then supplying fluid through the catheter until a desired degree of inflation is reached. The valve structure may be designed for partial and/or complete valve closure, as discussed above. After the catheter's distal end has been carried into the larger-diameter branch vessel, the wire may be further manipulated to open the valve structure, and/or fluid can be withdrawn from the catheter, to deflate the balloon.

Following the path illustrated in Figure 8, the next branch point encountered is at 136, where the vessel path follows the smaller-diameter branch vessel 148, as illustrated in detail in Figure 8. Here the balloon is in a preferably non-inflated state, to reduce the tendency of the catheter end to be carried in to the larger-diameter vessel. At this branch point, the guidewire is torqued to orient the wire's bent tip in the direction of vessel 148, and the wire is then advanced, preferably independently of the catheter, into the smaller vessel. The catheter may now be threaded along the guidewire, and the device advanced as a unit until the next branch point is reached. Further catheter advance is achieved by guidewire or balloon-flow guidance, as above. It will be appreciated that the guidewire may be used to advance the catheter through branch points in which the selected path may follow either a larger or smaller-diameter branch vessel. That is, balloon-assisted flow guidance need not be used for accessing a larger-diameter branch vessel. Of course, with smaller-diameter branch vessels, the guidewire must generally be employed.

The balloon catheter may also be used to dilate a constricted vessel, particularly, where the constriction is encountered along a vessel path, and makes advance along the path more difficult. This is done simply by locating the balloon at a region of spastic constriction, and manipulating the device to inflate the balloon.

Once the target site is reached, the guidewire may be removed in order to open the inner lumen of the catheter, and thereby increase the rate of supply to the target site of a therapeutic, vasoocclusive, and/or radio-opaque material. Alternatively, where the purpose of the targeting is to dilate the vessels or ducts at the target site, for example, to widen the lumen of a narrowed target vessel, either for angioplasty or spasm relaxation, the catheter device may be manipulated to inflate and deflate the balloon repeatedly. As indicated above, it may be advantageous to provide for complete valve closure at a selected guidewire position, to allow for greater balloon inflation pressure.

From the foregoing, it will be appreciated how various objects and features of the present invention are met. The catheter device takes advantage of both guidewire and balloon-flow guidance for movement along small-diameter tortuous vessel or duct paths, to allow more efficient and in some cases more versatile catheter targeting to a tissue site.

The balloon in the device can also be used for relaxing spasmodic vessel walls, and for vessel wall therapies, such as plaque compression requiring balloon inflation. In this application, the catheter allows access to tortuous-path soft tissue target sites, and the ability to inflate the balloon to moderately high pressures, in contrast to prior-art slow-leak balloon catheters. Alternatively, where the catheter device is used to deliver a fluid agent to the target site, the guidewire can be withdrawn to increase the lumen cross section.

It will be appreciated from Figure 2 that the guidewire, when provided with a radial enlargement, can also be advanced against the catheter aperture to maintain the flexible balloon in an uninflated, somewhat stretched condition. The guidewire and catheter can then be advanced as a unit along a vessel path, overcoming the problem of balloon buckling due to its inherent flexibility.

## Claims

1. A catheter device for accessing an internal tissue site along a vessel path from an external body access site, comprising: a catheter (12) having an inner lumen (15) extending between proximal and distal ends (16,18), an inflatable balloon (26) disposed adjacent the distal end (18), a communication being provided between the catheter lumen (15) and the interior of the balloon (26), and an aperture (30) disposed distally to said communication such that when the aperture (30) is blocked, fluid supplied through the lumen (15) is forced into the balloon (26), and a guidewire (36) having proximal and distal ends and carried in said catheter (12) for axial sliding movement therein, means being provided for at least partly closing said aperture (30) to permit the catheter balloon (26) to be inflated by supplying fluid through the catheter lumen (15), characterised in that said catheter aperture (30) is defined by an annular ring (28) mounted in the distal end of the catheter, and in that the means for at least partly closing said aperture comprises said guidewire (36) which is designed to block, partially or completely, the aperture (30) at one or more selected guidewire positions in said catheter (12).

2. The device of claim 1, wherein said catheter aperture (30) is dimensioned to receive a distal end segment of the guidewire (36) freely therethrough, with a clearance which is effective partially to block the aperture, when the wire is advanced through the ring.

3. The device of claim 1, wherein the guidewire includes a radial enlargement (43 or 48) effective to completely block said aperture (30) at a selected guidewire position within the catheter.

4. The device of claim 2, wherein the catheter (12) is formed of a tube (14) which has an intermediate inflatable section (26) forming said balloon, said ring (28) is positioned within the tube, distal to said inflatable section.

5. The device of claim 4, wherein the guidewire includes a radial enlargement (41) effective to completely block said aperture (30) at a selected guidewire position within the catheter.

6. The device of claim 2, wherein the catheter (12) is formed of a tube (74) having an inflatable distal end section which forms said balloon and said ring is positioned within the inflatable end section.

7. The device of any preceding claim, wherein the aperture (30) has an inner diameter than is 40% to 80% of the diameter of the inner lumen (15).

8. The device of any preceding claim, which further includes means (38) attached to the proximal end of the guidewire (36) for torquing the wire along its axis, to orient the distal end (42) of the wire in a selected direction.

## Patentansprüche

1. Kathetervorrichtung für den Zugang zu einer inneren Gewebestelle entlang einer Adernbahn ausgehend von einer äußeren Körperzugangsstelle, mit: einem Katheter (12) mit einem inneren Lumen (15), das sich zwischen proximalen und distalen Enden (16, 18) erstreckt, einem aufblasbaren Ballon (26), der sich nahe dem distalen Ende (18) befindet, einer Verbindung, die zwischen dem Katheterlumen (15) und dem Inneren des Ballons (26) vorgesehen ist, und einer Öffnung (30), die distal zu der Verbindung derart angeordnet ist, daß, wenn die Öffnung (30) blockiert ist, das durch das Lumen (15) zugeführte Fluid in den Ballon (26) gedrückt wird, und mit einem Führungsdraht (36), der proximale und distale Enden aufweist und in dem Katheter (12) so getragen wird, daß er sich darin axial gleitend bewegen kann, Mitteln, die dazu vorgesehen sind, die Öffnung (30) zumindest teilweise zu schließen, um das Aufblasen des Katheterballons (26) durch Zufuhr von Fluid durch das Katheterlumen (15) zuzulassen, dadurch gekennzeichnet, daß die Katheteröffnung (30) von einem ringförmigen Ring (28) begrenzt wird, der an dem distalen Ende des Katheters angebracht ist, und daß das Mittel zum zumindest teilweise Schließen der Öffnung den Führungsdraht (36) umfaßt, der so ausgelegt ist, daß er die Öffnung (30) an einer oder mehreren ausgewählten Führungsdrahtpositionen in dem Katheter (12) teilweise oder ganz blockiert.

2. Vorrichtung nach Anspruch 1, bei der die Katheteröffnung (30) so dimensioniert ist, daß sie ein distales Endsegment des Führungsdrahts (36) frei dort hindurch mit einem Zwischenraum aufnehmen kann, der dahingehend wirkt, die Öffnung teilweise zu blockieren, wenn der Draht durch den Ring vorwärtsbewegt wird.

3. Vorrichtung nach Anspruch 1, bei der der Führungsdraht eine radiale Vergrößerung (43 oder 48) aufweist, die dahingehend wirkt, die Öffnung (30) an einer ausgewählten Führungsdrahtposition in dem Katheter vollständig zu blockieren.

4. Vorrichtung nach Anspruch 2, bei der der Katheter (12) aus einem Schlauch (14) gebildet wird, der einen aufblasbaren Zwischenabschnitt (26) aufweist, der den Ballon bildet, wobei der Ring (28) in dem Schlauch distal zu dem aufblasbaren Abschnitt positioniert ist.

5. Vorrichtung nach Anspruch 4, bei der der Führungsdraht eine radiale Vergrößerung (41) umfaßt, die dahingehend wirkt, die Öffnung (30) an einer ausgewählten Führungsdrahtposition in dem Katheter vollständig zu blockieren.

6. Vorrichtung nach Anspruch 2, bei der der Katheter (12) aus einem Schlauch (74) gebildet wird, der einen aufblasbaren distalen Endabschnitt aufweist, der den Ballon bildet, und bei der der Ring in dem aufblasbaren Endabschnitt positioniert ist.

7. Vorrichtung nach einem der vorhergehenden Ansprüche, bei der die Öffnung einen Innendurchmesser von etwa 40% bis 80% des Durchmessers des inneren Lumen (15) aufweist.

8. Vorrichtung nach einem der vorhergehenden Ansprüche, die desweiteren Mittel (38) umfaßt, die an dem proximalen Ende des Führungsdrahts (36) angebracht sind, um den Draht entlang seiner Achse zu drehen, damit das distale Ende (42) des Drahtes in eine ausgewählte Richtung orientiert werden kann.

## Revendications

1. Dispositif à cathéter pour accéder à un site tissulaire interne le long d'un vaisseau à partir d'un site d'accès extérieur au corps comprenant : un cathéter (12) possédant un conduit intérieur (15) s'étendant entre des extrémités proximale et distale (16,18), un ballon gonflable (26) disposé au voisinage de l'extrémité distale (18), une communication étant réalisée entre le conduit (15) du cathéter et l'intérieur du ballon (26), une ouverture (30) disposée à distance de cette communication de telle manière que lorsque l'ouverture (30) est bloquée, du fluide amené au travers du conduit (15) est forcé dans le ballon (26), et un fil de guidage (36) possédant des extrémités proximale et distale et étant supporté dans ledit cathéter (12) pour un mouvement coulissant axial à l'intérieur, des moyens étant prévus pour fermer au moins partiellement ladite ouverture (30) pour permettre au ballon (26) du cathéter d'être gonflé en amenant du fluide au travers du conduit (15) du cathéter, caractérisé par le fait que ladite ouverture (30) du cathéter est définie par une bague annulaire (28) montée dans l'extrémité distale du cathéter, et que les moyens pour fermer au moins partiellement ladite ouverture comprennent ledit fil de guidage (36) qui est conçu pour bloquer partiellement ou complètement l'ouverture (30) pour une ou plusieurs positions choisies du fil de guidage dans ledit cathéter (12).

2. Dispositif selon la revendication 1, dans lequel ladite ouverture (30) du cathéter est dimentionnée pour recevoir un segment d'extrémité distale du fil de guidage (36) librement à travers elle avec un jeu permettant de bloquer partiellement l'ouverture lorsque le fil est avancé à travers la bague.

3. Dispositif selon la revendication 1, dans lequel le fil de guidage comprend un élargissement radial (43 ou 48) apte à bloquer complètement ladite ouverture (30) pour une position choisie du fil de guidage dans le cathéter.

4. Dispositif selon la revendication 2, dans lequel le cathéter (12) est constitué d'un tube (14) qui possède une section gonflable intermédiaire (26) formant ledit ballon, et ladite bague (28) est positionnée dans le tube à distance de ladite section gonflable.

5. Dispositif selon la revendication 4, dans lequel le fil de guidage comprend un élargissement radial (41) apte à bloquer complètement ladite ouverture (30) pour une position choisie du fil de guidage dans le cathéter.

6. Dispositif selon la revendication 2, dans lequel le cathéter (12) est constitué par un tube (74) possédant une section d'extrémité distale gonflable qui constitue ledit ballon et ladite bague positionnée dans le section d'extrémité gonflable.

7. Dispositif selon l'une quelconque des revendications précédentes, dans lequel l'ouverture (30) possède un diamètre intérieur qui est de 40 à 80 % du diamètre du conduit intérieur (15).

8. Dispositif selon l'une quelconque des revendications précédentes, qui comprend en outre des moyens (38) fixés à l'extrémité proximale du fil de guidage (36) pour provoquer une torsion du fil autour de son axe pour orienter l'extrémité distale (42) du fil dans une direction choisie.
